# EUROPÄISCHE PATENTSCHRIFT

(11) **EP 3 870 116 B1**
(45) Veröffentlichungstag und Bekanntmachung des Hinweises auf die Patenterteilung: **28.02.2024**
(21) Anmeldenummer: 19790163.0
(22) Anmeldetag: 14.10.2019
(51) Int. Cl.: A61F 2/70, A61F 2/60, A61F 5/01

(54) **ORTHOPÄDIETECHNISCHES GELENK UND ORTHOPÄDIETECHNISCHE EINRICHTUNG**
ORTHOPAEDIC JOINT AND ORTHOPAEDIC DEVICE
ARTICULATION TECHNIQUE ORTHOPÉDIQUE ET MÉCANISME ORTHOPÉDIQUE

(30) Priorität: 23.10.2018 DE 102018126324
(43) Veröffentlichungstag der Anmeldung: 01.09.2021
(73) Patentinhaber: Otto Bock Healthcare Products GmbH, 1110 Wien (AT)
(72) Erfinder: MEJIA NINO, Juan Pablo, 1160 Wien (AT); REITER, Dominik, 4400 Steyr (AT); AUBERGER, Roland, 1140 Wien (AT); KALMAR, Janos, 1140 Wien (AT)
(74) Vertreter: Gramm, Lins & Partner Patent- und Rechtsanwälte PartGmbB
(86) Internationale Anmeldenummer: PCT/EP2019/077817
(87) Internationale Veröffentlichungsnummer: WO 2020/083696

(56) Entgegenhaltungen:
- JP-A- 2016 174 681
- US-A1- 2007 038 168
- US-A1- 2010 198 362
- US-A1- 2010 298 746
- US-A1- 2011 009 788

## Beschreibung

Die Erfindung betrifft ein orthopädietechnisches Gelenk mit einem ersten Bauteil, einem zweiten Bauteil, das um eine Schwenkachse schwenkbar an dem ersten Bauteil angeordnet ist und einem Aktuator zum Verschwenken des ersten Bauteiles relativ zu dem zweiten Bauteil in zumindest einer Richtung, wobei der Aktuator lösbar an einer medialen oder lateralen Seite an dem ersten Bauteil oder dem zweiten Bauteil angeordnet ist und ein erstes Kupplungselement aufweist, das mit einem zweiten Kupplungselement des zweiten Bauteiles zusammenwirkt. Die Erfindung betrifft zudem eine orthopädietechnische Einrichtung mit wenigstens einem derartigen Gelenk.

Ein ähnliches Gelenk ist beispielsweise aus der US 2011/009788 A1, der JP 2016/174861 A und der US 2007/038168 A1 bekannt. Eine andere Ausgestaltung eines orthopädietechnischen Gelenkes ist beispielsweise aus der WO 2016/094413 A1 bekannt.

Orthopädietechnische Gelenke, beispielsweise Prothesenkniegelenke, sind aus dem Stand der Technik seit langem bekannt und werden in unterschiedlichsten Ausführungsformen angeboten. Sogenannte passive orthopädietechnische Gelenke verfügen dabei nicht über einen eigenen Antrieb, sondern lediglich über einen Dämpfer, mit dem eine Schwenkbewegung der beiden Bauteile relativ zueinander gedämpft werden kann. Durch den Dämpfer kann ein mehr oder weniger natürliches Gangbild beim Träger einer Prothese mit einem derartigen Kniegelenk erreicht werden. Moderne passive orthopädietechnische Gelenke können so ausgebildet sein, dass die Dämpfung einstellbar ist. So kann beispielsweise in einem Hydraulikschaltkreis ein Drosselventil geöffnet oder geschlossen werden, um eine Dämpfung, die ein Hydraulikdämpfer erzeugt, zu senken oder zu erhöhen. Um dies zum richtigen Zeitpunkt beispielsweise in einem Gangzyklus, zu erreichen, kann das orthopädietechnische Gelenk über wenigstens einen Sensor verfügen, dessen Messwerte von einer elektronischen Datenverarbeitungseinrichtung, die die Aufgabe einer elektronischen Steuerung übernimmt, ausgewertet und zur Steuerung des Dämpfers verwendet werden. Obwohl derartige Gelenke oftmals eine eigene Stromversorgung benötigen, handelt es sich um passive orthopädietechnische Gelenke. Ein Dämpfer ist auch bei passiven orthopädietechnischen Gelenken jedoch nicht zwingend nötig. Es gibt auch passive Gelenke, die ohne Dämpfer auskommen, so dass die beiden Bauteile frei schwingen können.

Aktive orthopädietechnische Gelenke verfügen über einen eigenen Aktuator, beispielsweise in Form eines Elektromotors oder einer Pumpe, durch die ein orthopädietechnisches Gelenk aktiv betätigt, also durch Betätigen des Aktuator das erste Bauteil relativ zum zweiten Bauteil um die Schwenkachse verschwenkt werden kann. Der Aktuator kann zusätzlich oder alternativ auch einer Verschwenkung als Dämpfer entgegenwirken, was beispielsweise in einem künstlichen Kniegelenk beim Berg-ab-Gehen von Vorteil ist.

Die Möglichkeit, ein orthopädietechnisches Gelenk aktiv zu bewegen ist für den Benutzer oftmals jedoch nur in bestimmten Bewegungssituationen, Stadien einer Therapie oder Trainingszuständen, nötig oder von Vorteil. Es hat sich dabei als vorteilhaft herausgestellt, wenn der Aktuator zuschaltbar und abschaltbar ist. Der Aktuator kann folglich in einen aktiven Zustand gebracht werden, in der er eingerichtet ist, das erste Bauteil relativ zu dem zweiten Bauteil in wenigstens eine Richtung zu verschwenken. Der Aktuator ist jedoch zusätzlich in einen passiven Zustand bringbar, in der er keinen Einfluss auf die Bewegung des orthopädietechnischen Gelenkes hat, so dass das aktive orthopädietechnische Gelenk in diesem Zustand des Aktuator zu einem passiven orthopädietechnischen Gelenk wird.

Nachteilig ist jedoch, dass bei derartigen Gelenken aus dem Stand der Technik der Aktuator ein erhöhtes Gewicht mit sich bringt und zudem am Unterschenkel angeordnet ist, so dass auch ein erhöhtes Drehmoment nötig ist, um das erste Bauteil relativ zu dem zweiten Bauteil um die Schwenkachse zu verschwenken. Das gesamte orthopädietechnische Gelenk ist konstruktiv aufwendig und damit kostenintensiv und zudem schwer.

Der Erfindung liegt die Aufgabe zugrunde, die Nachteile aus dem Stand der Technik zu beheben oder zumindest zu mindern.

Die Erfindung löst die gestellte Aufgabe durch ein orthopädietechnisches Gelenk gemäß dem Oberbegriff des Anspruchs 1, das sich dadurch auszeichnet, dass das erste Kupplungselement ein Mitnehmer ist, der in einen ersten Zustand, in dem er mit dem zweiten Kupplungselement derart zusammenwirkt, dass der Aktuator das erste Bauteil relativ zu dem zweiten Bauteil in eine erste Richtung verschwenkt, und in einen zweiten Zustand, in dem er keinen Einfluss auf eine Bewegung der beiden Bauteile zueinander hat, und in einen dritten Zustand bringbar ist, in dem er mit dem zweiten Kupplungselement derart zusammenwirkt, dass der Aktuator das erste Bauteil relativ zu dem zweiten Bauteil in eine zweite Richtung verschwenkt

Durch diese Ausgestaltung werden mehrere Vorteile erreicht. Durch die lösbare Anordnung des Aktuator kann für den Fall, dass der Aktuator nicht verwendet werden soll, dieser einfach entfernt werden, so dass bei dem dann entstehenden passiven orthopädietechnischen Gelenk kein zusätzliches Gewicht vorhanden ist, dass durch den nicht benötigen Aktuator hervorgerufen wird. Durch die Anordnung medial oder lateral des Gelenkes, also entlang der Schwenkachse versetzt, wird zudem kein zusätzliches Drehmoment oder Trägheitsmoment erzeugt, wenn das erste Bauteil relativ zu dem zweiten Bauteil um die Schwenkachse verschwenkt wird. Besonders vorteilhafterweise ist der Aktuator dabei sowohl an der medialen als auch an der lateralen Seite befestigbar. Dies ist beispielsweise bei der Verwendung des orthopädietechnischen Gelenks als Kniegelenk von Vorteil, da sowohl ein rechtes Kniegelenk als auch ein linkes Kniegelenk mit dem gleichen Aktuator ausgestattet werden kann. Ein passives Kniegelenk ist vorteilhafterweise symmetrisch zu einer Sagittalebene ausgebildet und verfügt sowohl auf der medialen als auch auf der lateralen Seite über entsprechende Kupplungsmittel, mit denen der Antrieb verbunden werden kann. Die Anzahl der benötigen Kopplungsadapter oder Aktuatorgehäuse oder Aktuator wird auf diese Weise stark reduziert.

Zusätzlich wird durch die erfindungsgemäße Ausgestaltung erreicht, dass unterschiedliche Aktuatoren, die sich beispielsweise in der Motorleistung unterscheiden können, an dem ersten Bauteil oder dem zweiten Bauteil angeordnet werden können. Auf diese Weise kann beispielsweise einem Trainingsfortschritt Rechnung getragen werden. Im Laufe der Zeit kann sich beispielsweise die benötigte Motorleistung eines Patienten bei einem aktiven orthopädietechnischen Gelenk verringern, wenn sich beispielsweise Trainingserfolge einstellen. Mit der erfindungsgemäßen Ausgestaltung des orthopädietechnischen Gelenkes kann ein Aktuator einfach vom ersten Bauteil oder vom zweiten Bauteil entfernt werden und durch einen anderen Aktuator mit geringerer oder gegebenenfalls erhöhter Motorleistung ersetzt werden. Dies ist beispielsweise auch von Vorteil, wenn ein Prothesenträger, in dessen Prothese wenigstens ein erfindungsgemäßes orthopädietechnisches Gelenk verwendet wird, schwimmen oder baden möchte. In diesem Fall kann der Aktuator, der beispielsweise ein Elektromotor enthalten kann, einfach vom ersten Bauteil oder vom zweiten Bauteil demontiert und das orthopädietechnische Gelenk im Wasser als passives orthopädietechnisches Gelenk verwendet werden.

Vorzugweise verfügt das orthopädietechnische Gelenk über wenigstens einen Dämpfer, der eingerichtet und angeordnet ist, ein Verschwenken des ersten Bauteiles relativ zu dem zweiten Bauteil zu dämpfen. Dabei ist die durch den Dämpfer hervorgerufene Dämpfung vorzugsweise einstellbar. Besonders bevorzugt ist die Dämpfung während des Verschwenkens des ersten Bauteils relativ zu dem zweiten Bauteil einstellbar.

In einer bevorzugten Ausgestaltung verläuft eine Abtriebswelle des Aktuators parallel zur Schwenkachse. Besonders bevorzugt verläuft die Abtriebswelle koaxial zur Schwenkachse. Dies ermöglicht eine Anordnung des Aktuators seitlich, also medial oder lateral, neben dem ersten Bauteil und/oder dem zweiten Bauteil, durch die die Schwenkachse verläuft. Die Schwenkachse ist dabei nicht notwendigerweise ein separates Bauteil. Sie kann auch als theoretische Schwenkachse, um die eine Verschwenkung des ersten Bauteils relativ zum zweiten Bauteil stattfindet, ausgebildet sein. Durch die Anordnung des Aktuators entlang dieser Schwenkachse wird das durch das Gewicht des Antriebs hervorgerufene zusätzliche Drehmoment, das aufgebracht werden muss, um das erste Bauteil relativ zum zweiten Bauteil zu verschwenken, minimiert und das Trägheitsmoment kaum verändert. In der optimalen Anordnung wird kein zusätzliches Drehmoment nötig und das Trägheitsmoment nicht verändert.

Es kann jedoch auch von Vorteil sein, wenn die Abtriebswelle des Aktuators nicht parallel zu der Schwenkachse verläuft. In diesem Fall befindet sich zwischen der Richtung der Abtriebswelle und der Schwenkachse vorzugsweise ein rechter Winkel.

Vorzugsweise verfügt der Aktuator über einen Antrieb beispielsweise in Form eines Motor mit einem Getriebe. Beide sind vorteilhafterweise als jeweils ein Modul ausgebildet, die voneinander lösbar sind. Bevorzugt wird das Getriebe an dem ersten Bauteil oder dem zweiten Bauteil angeordnet. Der Motor ist vorzugsweise am Getriebe angeordnet. Durch die modulare Ausgestaltung von Motor und Getriebe können mit begrenzter Auswahl an Motoren und/oder Getrieben eine Vielzahl von Kombinationen aus Getriebe und Motor bereit gestellt werden, um den individuellen Bedürfnissen des Patienten Rechnung zu tragen.

Vorzugsweise weist der Aktuator ein elastisches Element, insbesondere eine Feder, beispielsweise eine Drehfeder auf und/oder der Aktuator weist einen seriell elastischen Aktuator und/oder einen parallel elastischen Aktuator auf.

Bevorzugt ist das Getriebe selbsthemmend.

Besonders bevorzugt verfügt der Aktuator über ein Batteriemodul, das vorteilhafterweise lösbar an dem Motor oder an dem Getriebe angeordnet ist. In der bevorzugten Ausgestaltung ist das Getriebe am ersten Bauteil oder am zweiten Bauteil angeordnet, während der Motor am Getriebe und das Batteriemodul am Motor angeordnet ist. Dies hat den großen Vorteil, dass das Batteriemodul beispielsweise bei einer leeren Batterie oder einem leeren Akku einfach und mit wenig Montageaufwand durch ein anderes Batteriemodul ersetzt werden kann. Das Batteriemodul selbst kann vorteilhafterweise über eine separate Ladestation geladen werden, ohne dass es dazu in Kontakt mit dem Motor, dem Getriebe oder einem anderen Bauteil des orthopädietechnischen Gelenkes stehen muss. Verfügt ein Träger einer Prothese, in der ein orthopädietechnisches Gelenk der hier beschriebenen Art vorhanden ist, über zwei Batteriemodule, kann eines der Batteriemodule geladen werden, während das andere Batteriemodul den Motor mit elektrischer Energie versorgt. Der Motor ist vorteilhafterweise ein Elektromotor, beispielsweise ein Rotationsmotor.

Erfindungsgemäß verfügt der Aktuator über ein erstes Kupplungselement, das mit einem zweiten Kupplungselement zusammen wirkt, das am ersten Bauteil oder am zweiten Bauteil angeordnet ist. Dabei kann es sich beispielsweise über eine Stirnverzahnung, eine magnetische Kupplung, eine Umfangsverzahnung mit Klauenkupplung oder eine andere Art der Kupplung zwischen den beiden Kupplungselementen handeln.

Erfindungsgemäß ist das erste Kupplungselement ein Mitnehmer, der in einen ersten Zustand und in einen zweiten Zustand bringbar ist. In dem ersten Zustand wirkt er so mit dem zweiten Kupplungselement zusammen, dass der Aktuator das erste Bauteil relativ zu dem zweiten Bauteil in eine erste Richtung verschwenkt. Dazu kann der Mitnehmer beispielsweise an einer Seite am zweiten Kupplungselement des ersten Bauteils oder des zweiten Bauteils anliegen. Wird nun der Aktuator betätigt, wird der Mitnehmer um die Schwenkachse herum gedreht, und bewegt auf diese Weise auch das zweite Kupplungselement. Dieses ist drehfest mit dem der beiden Bauteile verbunden, mit dem der Aktuator nicht verbunden ist. Ist der Aktuator beispielsweise mit dem ersten Bauteil verbunden, handelt es sich bei dem zweiten Kupplungselement um ein Kupplungselement des zweiten Bauteils und umgekehrt. Durch Verdrehen des Mitnehmers um die Schwenkachse herum wird folglich auch das zweite Kupplungselement um die Schwenkachse herum bewegt und es kommt zu einem Verschwenken des ersten Bauteils relativ zum zweiten Bauteil um die gemeinsame Schwenkachse.

In dem zweiten Zustand des Mitnehmers hat dieser keinen Einfluss auf eine Bewegung der beiden Bauteile relativ zueinander. Er hat bevorzugt keinen Kontakt zum zweiten Kupplungselement. In diesem Sinne handelt es sich bei dem orthopädietechnischen Gelenk folglich um ein passives orthopädietechnisches Gelenk, da der Aktuator zwar vorhanden, jedoch nicht eingerichtet ist, eine Verschwenkung der beiden Bauteile relativ zueinander hervorzurufen.

Erfindungsgemäß ist der Mitnehmer zudem in einen dritten Zustand bringbar, in der er mit dem zweiten Kupplungselement derart zusammenwirkt, dass der Aktuator das erste Bauteil relativ zu dem zweiten Bauteil in eine zweite Richtung verschwenkt. Diese zweite Richtung ist vorteilhafterweise der ersten Richtung entgegengesetzt.

Besonders bevorzugt kann der Mitnehmer auch so ausgebildet sein, dass er in einem vierten Zustand angeordnet werden kann, in der er das erste Bauteil relativ zum zweiten Bauteil in beide Richtungen verschwenken kann, je nachdem, in welcher Richtung der Aktuator betrieben wird.

Die verschiedenen Zustände, in die der Mitnehmer bringbar ist, können beispielsweise unterschiedliche Positionen und/oder Orientierungen des Mitnehmers relativ zu dem Bauteil, an dem er angeordnet ist, sein.

Vorzugsweise ist eine Schnittstelle zwischen dem Aktuator und dem ersten Bauteil oder dem zweiten Bauteil wasserdicht. Auf diese Weise kann das orthopädietechnische Gelenk auch unter Wasser, beispielsweise beim Schwimmen, im Bad oder unter der Dusche verwendet werden.

Vorzugsweise ist das Gelenk ein Orthosengelenk, insbesondere ein Orthesenkniegelenk oder ein Orthesenhüftgelenk, oder ein Prothesengelenk, insbesondere ein Prothesenkniegelenk.

In einer bevorzugten Ausgestaltung ist der Aktuator in einen aktiven Zustand und einen passiven Zustand bringbar. Im aktiven Zustand ist der Aktuator und insbesondere dessen Motor eingerichtet, eine Kraft auf das erste und/oder das zweite Bauteil aufzubringen, um so das erste Bauteil relativ zu dem zweiten Bauteil in zumindest eine Richtung zu verschwenken. Im passiven Zustand ist der Aktuator nicht dazu in der Lage, eine solche Kraft aufzubringen. Auf diese Weise ist es möglich, die Energieversorgung des Aktuator, beispielsweise einen Akku für einen Elektromotor, zu schonen, wenn der Träger einer orthopädietechnischen Einrichtung, die das orthopädietechnische Gelenk enthält, die Unterstützung durch den Aktuator nicht oder zumindest nicht dauerhaft benötigt.

Bevorzugt wird die Dämpfung reduziert, wenn der Aktuator in den aktiven Zustand gebracht wird. Besonders bevorzugt wird sie soweit reduziert, wie dies durch den vorhandenen Dämpfer möglich ist. So wird beispielswiese ein Drosselventil, das beispielsweise in einem hydraulischen Dämpfer angeordnet ist, soweit es geht geöffnet, um die Dämpfung soweit es geht zu reduzieren.. In einer bevorzugten Ausgestaltung geschieht die Reduzierung der Dämpfung, indem der Aktuator in den aktiven Zustand gebracht wird. Dies kann beispielsweise erreicht werden, indem das Betätigungselement, das betätigt wird, um den Aktuator in den aktiven Zustand zu bringen, auch die Dämpfung beeinflusst, indem beispielsweise ein Hydraulikventil oder Pneumatikventil des Dämpfers geöffnet und so ein Strömungswiderstand reduziert oder entfernt wird.

Vorzugsweise ist der Aktuator durch Betätigen eines Betätigungselementes von dem passiven Zustand in den aktiven Zustand und/oder vom aktiven Zustand in den passiven Zustand bringbar. Das Betätigungselement kann beispielsweise direkt am orthopädietechnischen Gelenk, vorzugsweise an dessen Aktuator, besonders bevorzugt an dessen Motor, angeordnet sein. Es kann ein Schalter, beispielsweise ein Kipp- oder Drehschalter, ein Druckknopf, ein Hebel oder ein sonstiges mechanisches Element sein. Alternativ oder zusätzlich dazu kann auch beispielsweise ein Zugelement, beispielsweise in Form eines Seilzuges oder Kabels vorhanden sein, das als Betätigungselement dient. Wird eine Zugkraft auf dieses Zugelement ausgeübt, wird das Betätigungselement dadurch betätigt. Derartige Seilzugsysteme sind beispielsweise bekannt, um Handprothesen zu betätigen. Der Seilzug, der als Betätigungselement dient, wird in diesem Fall oftmals an der unversorgten Schulter angeordnet, sodass über die Bewegung dieser Schulter die Handprothese geöffnet oder geschlossen werden kann. Ähnliches ist auch bei einem orthopädietechnischen Gelenk der vorliegenden Art möglich. Wird das orthopädietechnische Gelenk beispielsweise in einer Orthese oder Prothese für die obere Extremität angeordnet, kann das Zugelement beispielsweise an der Schulter der jeweils unversorgten Extremität angeordnet werden.

Alternativ oder zusätzlich zu diesen mechanischen Betätigungselementen ist vorzugsweise ein elektronisches Betätigungselement vorgesehen. Dieses kann beispielsweise ein auf einem Display des orthopädietechnischen Gelenks dargestelltes Element sein, sofern dieses Display beispielsweise als Touchpad oder Touchscreen ausgebildet ist. Besonders bevorzugt ist das elektronische Betätigungselement jedoch nicht direkt am orthopädietechnischen Gelenk, sondern beispielsweise auf einem externen Gerät angeordnet. Besonders bevorzugt ist dabei die Integration des Betätigungselementes in eine App oder ein Computerprogrammprodukt, das auf einem externen elektronischen Datenverarbeitungsgerät, beispielsweise einem PC, einem Laptop oder insbesondere einem Smartphone, installiert und ausgeführt wird. Über eine Betätigung eines Betätigungselementes, beispielsweise in der App eines Smartphones, kann der Aktuator vom aktiven in den passiven Zustand oder umgekehrt gebracht werden. Auf diese Weise kann der Benutzer des orthopädietechnischen Gelenkes individuell entscheiden, ob er die Unterstützung des Aktuators momentan benötigt. Muss der Antrieb aus dem aktiven Zustand in den passiven Zustand oder umgekehrt gebracht werden, kann der Benutzer dies selbsttätig, schnell, einfach und sicher tun.

Befindet sich der Motor im aktiven Zustand, hat es sich als vorteilhaft herausgestellt, wenn er nicht dauerhaft die zum Verschwenken des ersten Bauteils relativ zum zweiten Bauteil nötige Kraft aufbringt. Ist das orthopädietechnische Gelenk beispielsweise ein Kniegelenk, kann es von Vorteil sein, die zum Verschwenken nötige Unterstützung des Aktuators beispielsweise nur in bestimmten Phasen eines Schrittzyklus aufzubringen. Dies kann beispielsweise die Schwungphase sein, in der das Kniegelenk durch die Unterstützung des Aktuators gestreckt, also extendiert wird. Vorteilhafterweise verfügt daher das orthopädietechnische Gelenk über eine elektronische Steuerung, insbesondere eine elektronische Datenverarbeitungseinrichtung, und wenigstens einen Sensor, der eingerichtet ist, eine für eine Bewegung des Gelenks relevante Größe zu detektieren. Diese Größe kann beispielsweise ein Gelenkwinkel des orthopädietechnischen Gelenkes, eine Änderung dieses Gelenkwinkels, eine Beschleunigung oder ein Momentenverlauf sein. Auf der Grundlage dieser Messwerte und der daraus im Sensor erzeugten Sensordaten kann die elektronische Steuerung den Aktuator steuern. Dies geschieht in Abhängigkeit der Sensordaten des wenigstens einen Sensors. Auf diese Weise wird die dem Aktuator zur Verfügung stehende Energie, die beispielsweise in einem elektrischen Energiespeicher, beispielsweise einem Akku, gespeichert ist, so verwendet, dass der Aktuator möglichst lange, also bei möglichst vielen Schrittzyklen, verwendet werden kann. Eine Energieverschwendung kann auf diese Weise vermieden oder zumindest verringert werden.

In einer bevorzugten Ausgestaltung kann der Aktuator im aktiven Zustand in mehreren Modi betrieben werden. Dieser Ausgestaltung liegt die Erkenntnis zugrunde, dass in Abhängigkeit des jeweiligen Bewegungszustandes der orthopädietechnischen Einrichtung, die mit dem orthopädietechnischen Gelenk der hier beschriebenen Art ausgestaltet ist, unterschiedliche Unterstützungsmodi benötigt werden oder vorteilhaft sind. So ist der Zeitpunkt innerhalb eines Schrittzyklus, zu dem der Antrieb eine Unterstützung bereitstellen sollte, davon abhängig, welche Bewegung der Träger der orthopädietechnischen Einrichtung ausführt. So kann beispielsweise über den wenigstens einen Sensor detektiert werden, ob der Träger beispielsweise entlang einer Ebene, entlang einer Schräge oder entlang einer Treppe geht. Unterschiedliche Ganggeschwindigkeiten können bestimmt werden. Zudem kann beispielsweise auch ein Gewicht bestimmt werden, das der Träger der orthopädietechnischen Einrichtung und des orthopädietechnischen Gelenkes trägt. In Abhängigkeit einzelner, mehrerer oder aller dieser Messdaten kann der jeweilige Betriebsmodus des Aktuators durch die elektronische Steuerung eingerichtet werden.

Alternativ oder zusätzlich ist es von Vorteil, wenn auch durch das Betätigen eines Betätigungselementes der Aktuator von einem Modus in einen anderen Modus gebracht werden kann. Dieses Betätigungselement kann dabei das gleiche Betätigungselement sein, durch dessen Betätigung der Antrieb auch vom passiven Zustand in den aktiven Zustand bringbar ist. Selbstverständlich ist es auch möglich, unterschiedliche Betätigungselemente vorzusehen.

Die Erfindung löst die gestellte Aufgabe zudem durch eine orthopädietechnische Einrichtung, insbesondere eine Orthese oder Prothese, mit wenigstens einem orthopädietechnischen Gelenk der hier beschriebenen Art.

Mit Hilfe der beigefügten Figuren wird nachfolgend ein Ausführungsbeispiel der vorliegenden Erfindung näher erläutert. Es zeigt
Figur 1 - die schematische Darstellung eines Gelenkes gemäß einem ersten Ausführungsbeispiel der vorliegenden Erfindung,
Figur 2 - die schematische Darstellung der Ankopplung des Aktuators in einer Explosionsdarstellung und
Figur 3 - drei unterschiedliche Zustände eines Kopplungselementes in einer schematischen Darstellung.

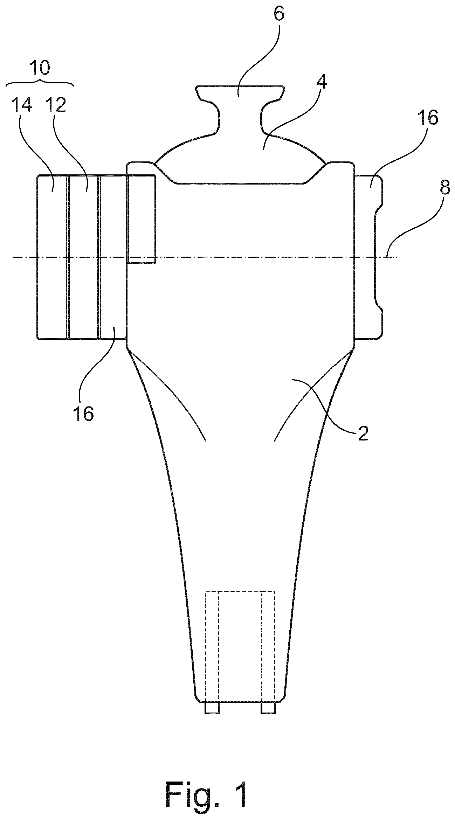

Figur 1 zeigt ein orthopädietechnisches Gelenk in Form eines künstlichen Kniegelenkes, das ein erstes Bauteil 2 und ein daran angeordnetes zweites Bauteil 4 aufweist. Im gezeigten Ausführungsbeispiel kann am unteren Ende des ersten Bauteiles 2 ein Unterschenkelelement angeordnet werden, während am oberen Ende des zweiten Bauteiles 4 ein Pyramidenadapter 6 angeordnet ist, an dem weitere Prothesenkomponenten, beispielsweise ein Oberschenkelschaft, angeordnet werden können.

Das Gelenk verfügt über einen Dämpfer, der nicht dargestellt ist. Das erste Bauteil 2 ist relativ zum zweiten Bauteil 4 um eine Schwenkachse 8 schwenkbar gelagert. Bei dem Gelenk handelt es sich um ein aktives Gelenk, das über einen Aktuator 10 verfügt, der im gezeigten Ausführungsbeispiel ein Getriebe 12 und einen Motor 14 aufweist. Das Getriebe 12 ist an einem Befestigungselement 16 angeordnet, und leicht lösbar ausgebildet. Der Motor 14 ist leicht lösbar am Getriebe 12 angeordnet, sodass beispielsweise der Motor 14 allein oder in Kombination mit dem Getriebe 12 gegen beispielsweise eine andere Ausgestaltung von Getriebe und Motor ausgewechselt werden kann.

Das Gelenk ist symmetrisch ausgebildet. Während in Figur 1 auf der linken Seite an dem Befestigungselement 16 ein Getriebe 12 und ein Motor 14 angeordnet sind, ist auch die rechte Seite mit einem entsprechenden Befestigungselement 16 ausgestaltet, ohne dass die Bauteile angeordnet sind.

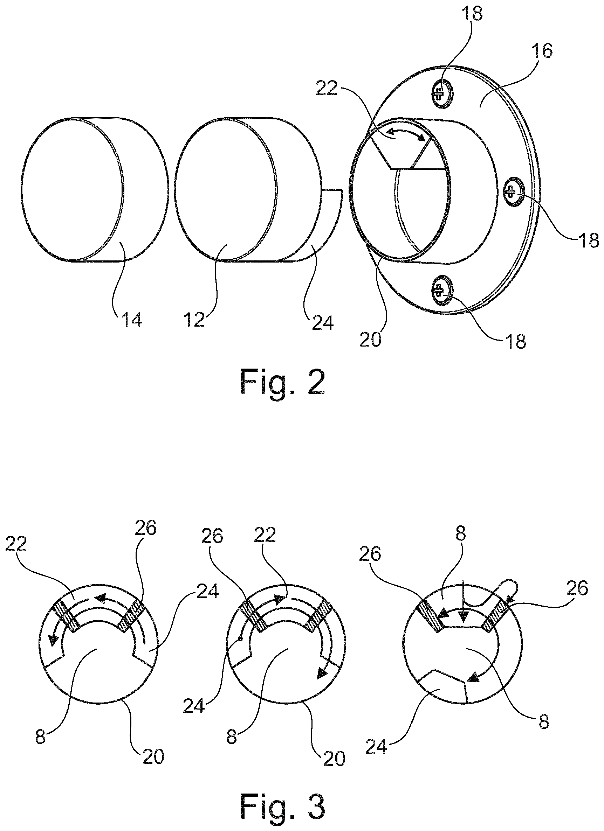

Figur 2 zeigt einen Ausschnitt in vergrößerter Explosionsdarstellung. Man erkennt zunächst das Befestigungselement 16, das durch Schrauben 18 am ersten Bauteil 2 drehfest angeordnet ist. Es verfügt über einen ringförmigen Vorsprung 20, innerhalb dessen ein zweites Kopplungselement 22 angeordnet ist, das drehfest mit dem zweiten Bauteil 4 verbunden ist. Am Getriebe 12 befindet sich ein erstes Kupplungselement 24, das im montierten Zustand, der in Figur 1 schematisch dargestellt ist, in den ringförmigen Vorsprung 20 hineinragt. Je nach Position und Orientierung des ersten Kupplungselementes 24 relativ zum zweiten Kupplungselement 22 können die unterschiedlichen Zustände der Kupplungselemente erreicht werden.

Dies ist in Figur 3 schematisch dargestellt. Gezeigt ist jeweils eine schematische Ansicht entlang der Schwenkachse 8. Man erkennt den ringförmigen Vorsprung 20, in dem sich jeweils im oberen Bereich das zweite Kupplungselement 22 befindet. In der linken Darstellung ist das erste Kupplungselement 24 so dargestellt, dass es, durch ein elastisches Dämpfungselement 26 getrennt, am zweiten Kupplungselement 22 anliegt. Wird nun der Motor 14 oder ein anderer Aktuator 10 so angetrieben, dass sich das erste Kupplungselement 24, das drehfest mit dem Getriebe 12 oder einem anderen Bauteil des Aktuators 10 verbunden ist, entgegen dem Uhrzeigersinn bewegt, wird auch das zweite Kupplungselement 22 bewegt und das Gelenk angetrieben. Wird der Aktuator 10 in die entgegengesetzte Richtung betrieben, sodass sich das erste Kupplungselement 24 im Uhrzeigersinn bewegt, findet keine Drehmomentübertragung statt, sodass das Gelenk nicht angetrieben wird.

Im mittleren Teil der Figur 3 ist eine andere Darstellung gezeigt. Auch hier befindet sich im oberen Teil das zweite Kupplungselement 22 innerhalb des ringförmigen Vorsprungs 20. Das erste Kupplungselement 24 befindet sich nun links vom zweiten Kupplungselement 22, auch von diesem durch ein elastisches Dämpfungselement 26 getrennt. Nun wird das zweite Kupplungselement 22 bewegt, wenn der Aktuator 10 so betrieben wird, dass sich das erste Kupplungselement 24 im Uhrzeigersinn bewegt.

In der rechten Darstellung von Figur 3 liegt das erste Kupplungselement 24 gar nicht an einem der elastischen Dämpfungselemente 26 an. Unabhängig davon, in welche Richtung das erste Kupplungselement 24 durch den Aktuator 10 bewegt wird, kommt es nicht zu einer Bewegung des zweiten Kupplungselements 22 und damit auch nicht zu einer Betätigung des Gelenkes.

### Bezugszeichenliste

- 2: erstes Bauteil
- 4: zweites Bauteil
- 6: Pyram idenadapter
- 8: Schwenkachse
- 10: Aktuator
- 12: Getriebe
- 14: Motor
- 16: Befestigungselement
- 18: Schraube
- 20: Vorsprung
- 22: zweites Kupplungselement
- 24: erstes Kupplungselement
- 26: elastisches Dämpfungselement

## Patentansprüche

1. Orthopädietechnisches Gelenk mit
• einem ersten Bauteil (2),
• einem zweiten Bauteil (4), das um eine Schwenkachse (8) schwenkbar an dem ersten Bauteil (2) angeordnet ist, und
• einem Aktuator (10) zum Verschwenken des ersten Bauteiles (2) relativ zu dem zweiten Bauteil (4) in zumindest einer Richtung,
wobei der Aktuator (10) lösbar an einer medialen oder lateralen Seite an dem ersten Bauteil (2) angeordnet ist und ein erstes Kupplungselement (24) aufweist, das mit einem zweiten Kupplungselement (22) des zweiten Bauteiles (4) zusammenwirkt **dadurch gekennzeichnet, dass** das erste Kupplungselement (24) ein Mitnehmer ist, der in einen ersten Zustand, in dem er mit dem zweiten Kupplungselement (22) derart zusammenwirkt, dass der Aktuator (10) das erste Bauteil (2) relativ zu dem zweiten Bauteil (4) in eine erste Richtung verschwenkt, und in einen zweiten Zustand, in dem er keinen Einfluss auf eine Bewegung der beiden Bauteile (2, 4) zueinander hat, und in einen dritten Zustand bringbar ist, in dem er mit dem zweiten Kupplungselement (22) derart zusammenwirkt, dass der Aktuator (10) das erste Bauteil (2) relativ zu dem zweiten Bauteil (4) in eine zweite Richtung verschwenkt.

2. Orthopädietechnisches Gelenk nach Anspruch 1, **dadurch gekennzeichnet, dass** das Gelenk wenigstens einen Dämpfer aufweist, der eingerichtet und angeordnet ist, ein Verschwenken des ersten Bauteiles (2) relativ zu dem zweiten Bauteil (4) zu dämpfen.

3. Orthopädietechnisches Gelenk nach Anspruch 2, **dadurch gekennzeichnet, dass** die von dem Dämpfer hervorgerufene Dämpfung, vorzugsweise während des Verschwenkens des ersten Bauteils (2) relativ zu dem zweiten Bauteil (4), einstellbar ist.

4. Orthopädietechnisches Gelenk nach Anspruch 1, 2 oder 3, **dadurch gekennzeichnet, dass** eine Abtriebswelle des Aktuators (10) parallel zu der Schwenkachse (8) verläuft.

5. Orthopädietechnisches Gelenk nach einem der vorstehenden Ansprüche, **dadurch gekennzeichnet, dass** der Aktuator (10) einen Motor (14) und ein Getriebe (12) aufweist, die vorzugsweise jeweils als ein Modul ausgebildet sind, die voneinander lösbar sind.

6. Orthopädietechnisches Gelenk nach einem der vorstehenden Ansprüche, **dadurch gekennzeichnet, dass** der Aktuator (10) ein elastisches Element, insbesondere eine Feder, beispielsweise eine Drehfeder aufweist und/oder einen seriell elastischen Aktuator und/oder einen parallel elastischen Aktuator aufweist.

7. Orthopädietechnisches Gelenk nach Anspruch 5 oder 6, **dadurch gekennzeichnet, dass** das Getriebe selbsthemmend ist.

8. Orthopädietechnisches Gelenk nach einem der vorstehenden Ansprüche, **dadurch gekennzeichnet, dass** der Aktuator (10) ein Batteriemodul aufweist, das vorzugsweise lösbar an dem Motor (14) oder dem Getriebe (12) angeordnet ist.

9. Orthopädietechnisches Gelenk nach einem der vorstehenden Ansprüche, **dadurch gekennzeichnet, dass** eine Schnittstelle zwischen dem Aktuator (10) und dem ersten Bauteil (2) oder dem zweiten Bauteil (4) wasserdicht ist.

10. Orthopädietechnisches Gelenk nach einem der vorstehenden Ansprüche, **dadurch gekennzeichnet, dass** das Gelenk ein Orthesengelenk, insbesondere ein Orthesenkniegelenk oder Orthesenhüftgelenk, oder ein Prothesengelenk, insbesondere ein Prothesenkniegelenk ist.

11. Orthopädietechnisches Gelenk nach einem der vorstehenden Ansprüche, **dadurch gekennzeichnet, dass** der Aktuator (10) in einen aktiven Zustand und in einen passiven Zustand bringbar ist, wobei vorzugsweise die Dämpfung reduziert wird, wenn der Aktuator (10) in den aktiven Zustand gebracht wird.

12. Orthopädietechnisches Gelenk nach Anspruch 11, **dadurch gekennzeichnet, dass** das Gelenk ein Betätigungselement aufweist und der Aktuator (10) durch Betätigen des Betätigungselementes, insbesondere eines an dem Gelenk, vorzugsweise an dem Aktuator (10), angeordneten Schalters, Druccknopfes oder Hebels, eines Seilzuges oder Kabels, von dem passiven Zustand in den aktiven Zustand und/oder umgekehrt bringbar ist.

13. Orthopädietechnisches Gelenk nach Anspruch 11 oder 12, **dadurch gekennzeichnet, dass** das Gelenk über eine elektronische Steuerung, insbesondere eine elektronische Datenverarbeitungseinrichtung, und wenigstens einen Sensor verfügt, der eingerichtet ist, eine für eine Bewegung des Gelenks relevante Größe zu detektieren, und die elektronische Steuerung eingerichtet ist, den Aktuator (10) in Abhängigkeit der Sensordaten des wenigstens einen Sensors zu steuern.

14. Orthopädietechnische Einrichtung, insbesondere Orthese oder Prothese, mit wenigstens einem orthopädietechnischen Gelenk nach einem der vorstehenden Ansprüche.

## Claims

1. An orthopedic joint with
• a first component (2),
• a second component (4) that is arranged on the first component (2) such that it can be swivelled about a swivel axis (8), and
• an actuator (10) for swivelling the first component (2) relative to the second component (4) in at least one direction,
wherein the actuator (10) is arranged on the first component (2) on a medial or lateral side such that it can be detached and comprises a first coupling element (24) which interacts with a second coupling element (22) of the second component (4), **characterized in that** the first coupling element (24) is a driver, which can be brought into a first state, in which it interacts with the second coupling element (22) in such a way that the actuator (10) swivels the first component (2) relative to the second component (4) in a first direction, and into a second state, in which it has no influence on a movement of the two components relative to one another, and into a third state, in which it interacts with the second coupling element (22) in such a way that the actuator (10) swivels the first component (2) relative to the second component (4) in a second direction.

2. The orthopedic joint according to claim 1, **characterized in that** the joint has at least one damper that is configured and arranged to damp a swivelling of the first component (2) relative to the second component (4).

3. The orthopedic joint according to claim 2, **characterized in that** the damping caused by the damper can be adjusted, preferably during the swivelling of the first component (2) relative to the second component (4).

4. The orthopedic joint according to claim 1, 2 or 3, **characterized in that** a drive shaft of the actuator (10) extends parallel to the swivel axis (8).

5. The orthopedic joint according to one of the preceding claims, **characterized in that** the actuator (10) comprises a motor (14) and a transmission (12), which each are preferably designed as one module, which can be detached from each other.

6. The orthopedic joint according to one of the preceding claims, **characterized in that** the actuator (10) features an elastic element, particularly a spring, such as a torsion spring, and/or a series elastic actuator and/or a parallel elastic actuator.

7. The orthopedic joint according to claim 5 or 6, **characterized in that** the transmission is self-locking.

8. The orthopedic joint according to one of the preceding claims, **characterized in that** the actuator (10) comprises a battery module which is preferably arranged on the motor (14) or transmission (12) such that it can be detached.

9. The orthopedic joint according to one of the preceding claims, **characterized in that** an interface between the actuator (10) and the first component (2) or the second component (4) is waterproof.

10. The orthopedic joint according to one of the preceding claims, **characterized in that** the joint is an orthotic joint, in particular an orthotic knee joint or an orthotic hip joint, or a prosthesis joint, in particular a prosthetic knee joint.

11. The orthopedic joint according to one of the preceding claims, **characterized in that** the actuator (10) can be brought into an active state and a passive state, wherein the damping is preferably reduced when the actuator (10) is brought into the active state.

12. The orthopedic joint according to claim 11, **characterized in that** the joint comprises an actuating element and the actuator (10) can be brought from the passive state into the active state and/or vice versa by actuating the actuating element, in particular a switch, button or lever, a cable pull or cable, arranged on the joint, preferably on the actuator (10).

13. The orthopedic joint according to claim 11 or 12, **characterized in that** the joint has an electronic control system, in particular an electronic data processing device, and at least one sensor which is set up to detect a variable relevant to a movement of the joint, and the electronic control system is configured to control the actuator (10) depending on the sensor data of the at least one sensor.

14. An orthopedic device, in particular an orthosis or a prosthesis, with at least one orthopedic joint according to one of the preceding claims.

## Revendications

1. Articulation orthopédique comprenant
• un premier composant (2),
• un deuxième composant (4) disposé sur le premier composant (2) de manière à pouvoir pivoter autour d'un axe de pivotement (8), et
• un actionneur (10) destiné à faire pivoter le premier composant (2) par rapport au deuxième composant (4) dans au moins une direction, l'actionneur (10) étant disposé de manière amovible sur un côté médial ou latéral sur le premier composant (2) et comprenant un premier élément de couplage (24) qui coopère avec un deuxième élément de couplage (22) du deuxième composant (4),
**caractérisée en ce que** le premier élément de couplage (24) est un entraîneur qui peut être mis dans un premier état, dans lequel il coopère avec le deuxième élément de couplage (22) de telle sorte que l'actionneur (10) fait pivoter le premier composant (2) par rapport au deuxième composant (4) dans une première direction, et dans un deuxième état, dans lequel il n'a aucune influence sur un mouvement des deux composants (2, 4) l'un par rapport à l'autre, et dans un troisième état, dans lequel il coopère avec le deuxième élément de couplage (22) de telle sorte que l'actionneur (10) fait pivoter le premier composant (2) par rapport au deuxième composant (4) dans une deuxième direction.

2. Articulation orthopédique selon la revendication 1,
**caractérisée en ce que** l'articulation comprend au moins un amortisseur qui est conçu et agencé pour amortir un pivotement du premier composant (2) par rapport au deuxième composant (4).

3. Articulation orthopédique selon la revendication 2,
**caractérisée en ce que** l'amortissement provoqué par l'amortisseur est réglable, de préférence pendant le pivotement du premier composant (2) par rapport au deuxième composant (4).

4. Articulation orthopédique selon la revendication 1, 2 ou 3,
**caractérisée en ce qu'**un arbre de sortie de l'actionneur (10) s'étend parallèlement à l'axe de pivotement (8).

5. Articulation orthopédique selon l'une des revendications précédentes,
**caractérisée en ce que** l'actionneur (10) comprend un moteur (14) et un mécanisme (12), de préférence conçus respectivement sous forme de modules pouvant être détachés l'un de l'autre.

6. Articulation orthopédique selon l'une des revendications précédentes,
**caractérisée en ce que** l'actionneur (10) comprend un élément élastique, en particulier un ressort, par exemple un ressort de torsion, et/ou comprend un actionneur élastique en série et/ou un actionneur élastique en parallèle.

7. Articulation orthopédique selon la revendication 5 ou 6,
**caractérisée en ce que** le mécanisme est autobloquant.

8. Articulation orthopédique selon l'une des revendications précédentes,
**caractérisée en ce que** l'actionneur (10) comprend un module de batterie qui est de préférence disposé de manière amovible sur le moteur (14) ou sur le mécanisme (12).

9. Articulation orthopédique selon l'une des revendications précédentes,
**caractérisée en ce qu'**une interface entre l'actionneur (10) et le premier composant (2) ou le deuxième composant (4) est étanche à l'eau.

10. Articulation orthopédique selon l'une des revendications précédentes,
**caractérisée en ce que** l'articulation est une articulation d'orthèse, en particulier une articulation d'orthèse de genou ou une articulation d'orthèse de hanche, ou une articulation de prothèse, en particulier une articulation de prothèse de genou.

11. Articulation orthopédique selon l'une des revendications précédentes,
**caractérisée en ce que** l'actionneur (10) peut être mis dans un état actif et dans un état passif, de préférence, l'amortissement étant réduit lorsque l'actionneur (10) est mis dans l'état actif.

12. Articulation orthopédique selon la revendication 11,
**caractérisée en ce que** l'articulation comprend un élément d'actionnement, et l'actionneur (10) peut être amené de l'état passif à l'état actif et/ou inversement par l'actionnement de l'élément d'actionnement, en particulier d'un interrupteur, d'un bouton-poussoir ou d'un levier, d'un câble Bowden ou d'un cordon, disposé sur l'articulation, de préférence sur l'actionneur (10).

13. Articulation orthopédique selon la revendication 11 ou 12,
**caractérisée en ce que** l'articulation dispose d'une commande électronique, en particulier d'un dispositif électronique de traitement de données, et d'au moins un capteur conçu pour détecter une grandeur pertinente pour un mouvement de l'articulation, et la commande électronique est conçue pour commander l'actionneur (10) en fonction des données de capteur dudit au moins un capteur.

14. Dispositif orthopédique, en particulier orthèse ou prothèse, comprenant au moins une articulation orthopédique selon l'une des revendications précédentes.
